# EUROPÄISCHE PATENTSCHRIFT

(11) **EP 0 596 344 B1**
(45) Veröffentlichungstag und Bekanntmachung des Hinweises auf die Patenterteilung: **28.01.1998**
(21) Anmeldenummer: 93117124.3
(22) Anmeldetag: 22.10.1993
(51) Int. Cl.: A61B 5/0416, A61B 5/0428

(54) **Anschlussvorrichtung für EKG-Elektroden**
Connector device for ECG electrodes
Dispositif de raccordement pour électrodes de l'électro-cardiogramme

(30) Priorität: 06.11.1992 DE 9215099 U
(43) Veröffentlichungstag der Anmeldung: 11.05.1994
(73) Patentinhaber: B. BRAUN MELSUNGEN AG, 34212 Melsungen (DE)
(72) Erfinder: Göbel, Udo, W-3508 Meg.-Kirchhof (DE); Pingel, Jörg, W-3501 Edm.-Besse (DE)
(74) Vertreter: Selting, Günther, Dipl.-Ing.

(56) Entgegenhaltungen:
- DE-A- 3 431 187
- US-A- 4 644 960
- BIOMEDIZINISCHE TECHNIK, Bd.35, Nr.11, November 1990, BERLIN,DE, Seiten 281 - 282 'Neues aus der Forschung und Industrie, ARBO-VS-Blockstecksystem-das EKG-Monitorkabel für alle Anforderungen,'

## Beschreibung

Die Erfindung betrifft eine Anschlußvorrichtung zum Verbinden mit einem Klemmadapter oder einem Druckknopfadapter oder einem Bananensteckeradapter einer herkömmlichen EKG-Patientenleitung eines EKG-Monitors.

Für die Aufzeichnung von EKGs werden von einer Vielzahl von Herstellern unterschiedliche Systeme zur Verfügung gestellt. Das zentrale Gerät bildet jeweils ein Monitor, der u.a. eine Elektronenstrahlröhre zur Sichtbarmachung der EKG-Signale aufweist. Zu dem Monitor gehört ein Patientenkabel, das durch einen Stecker mit dem Monitor verbunden wird und das mehrere Leitungen aufweist, die mit am Patientenkörper befestigten Elektroden verbunden werden. Bei der Ableitung von EKGs wird jeweils dasjenige Patientenkabel benutzt, das vom Hersteller des Monitors angeboten wird und dessen Stecker mit dem Gegenstecker des Monitors zusammenpaßt. Die Patientenkabel der einzelnen Hersteller unterscheiden sich nicht nur durch ihre Stecker, sondern auch in ihren elektrischen Eigenschaften sowie auch in der Anzahl der Leitungen. Das Patientenkabel, das zu einem bestimmten Monitor gehört, kann nicht in Verbindung mit einem anderen EKG-Monitor benutzt werden.

Neben der bisher erläuterten extrakorporalen EKG-Ableitung wird in zunehmendem Maße eine intraatriale EKG-Ableitung durchgeführt, bei der zwecks Lageverifizierung eines Cava-Katheters derselbe kurzzeitig in den Vorhof des Herzes des Patienten verlegt wird. Dieser Cava-Katheter hat einen Leiterdraht oder erhält eine leitende Flüssigkeit zur Übertragung der elektrischen Spannung vom Herzen an eine außerhalb des Körpers liegende Stelle. Um ein für extrakorporale EKG-Ableitungen bestimmtes System für die intraatriale EKG-Ableitung zu benutzen, wird eine der Leitungen des Patientenkabels mit dem leitenden Medium des Cava-Katheters verbunden, während die anderen Leitungen des Patientenkabels weiterhin mit den extrakorporalen Elektroden verbunden bleiben. Die Leitungen des Patientenkabels sind mit Konnektoren versehen, die auf an dem Patientenkörper anzubringende Haftelektroden aufgesteckt werden. Diese Aufstecktechnik, die im extrakorporalen Bereich keine Schwierigkeiten verursacht, ist jedoch dann problematisch, wenn der betreffende Konnektor, der sich für das Aufstecken an eine Haftelektrode eignet, mit dem Leiterdraht des Herzkatheters verbunden werden soll. Hierzu sind Konnektoren, die für Haftelektroden bestimmt sind, grundsätzlich ungeeignet. Der Leiterdraht eines Cava-Katheters erfordert nämlich eine berührungssichere Adaption. Dies bedeutet, daß sichergestellt sein muß, daß der Leiterdraht oder ein mit ihm verbundener Draht nicht mit der Hand berührt werden kann. Solche Berührungen können im Herzen nicht gewünschte Rhythmusstörungen bzw. Arrhythmien verursachen.

Aus dem Prospekt "CAVAFIX CERTODYN" der B. Braun Melsungen AG ist ein Cava-Katheter bekannt, zu dem ein EKG-Patientenkabel geliefert wird, das eingangsseitig drei Leitungen aufweist, die mit Konnektoren an extrakorporale Elektroden angeschlossen werden können. Zusätzlich ist eine Anschlußmöglichkeit für den Cava-Katheter vorhanden. Mit einem Umschalter kann anstelle der einen extrakorporalen Elektrode der Cava-Katheter auf den Ausgang des Patientenkabels umgeschaltet werden, der an den EKG-Monitor angeschlossen ist. Damit ist es möglich, eine einfache Umschaltung zwischen extrakorporalem Brustwand-EKG und intraatrialem EKG vorzunehmen. Nachteilig ist jedoch, daß dieses Patientenkabel in zahlreichen unterschiedlichen Ausführungsformen geliefert werden muß, die an die unterschiedlichen EKG-Monitore verschiedener Hersteller angepaßt sind. Dabei sind nicht nur unterschiedliche Steckerformen vorzusehen, sondern auch unterschiedliche Beschaltungen innerhalb des in dem Patientenkabel vorhandenen Verteilers. Wegen der immensen Vielzahl unterschiedlicher Monitorhersteller und Monitortypen ist es jedoch nicht möglich, zu einem Cava-Katheter ein flächendeckendes Kabelangebot zu schaffen, das eine sehr große Anzahl unterschiedlicher Arten von Patientenkabeln umfassen würde.

Der Erfindung liegt die Aufgabe zugrunde, eine Anschlußvorrichtung für EKG-Verbindungsadapter von EKG-Patientenleitungen zu schaffen, so daß die intraatriale EKG-Ableitung mühelos mittels der Anschlußvorrichtung durchgeführt werden kann, ohne die anliegende monitorspezifische EKG-Patientenleitung auszutauschen, wodurch ein aufwendiges Kabelprogramm umschaltbarer Patientenleitungen überflüssig wird.

Die Lösung dieser Aufgabe erfolgt erfindungsgemäß mit den im Anspruch 1 oder 2 angegebenen Merkmalen.

Die erfindungsgemäße Anschlußvorrichtung besteht aus einem Universal-Adapter, dessen Eingangsleitung entweder ein Adapter (z.B. Klemmadapter) ist, der an eine Brustwandelektrode angeschlossen werden kann, oder aber als Verbindungshebel ausgestaltet ist zum direkten Anschluß an den Cava-Katheter. Der Universal-Adapter weist mehrere unterschiedlich gestaltete, wahlweise zu benutzende Ausgänge auf, die auf Adapter unterschiedlichen Typs von Patientenkabeln abgestimmt sind. Damit ist es möglich, irgendeinen EKG-Monitor mit dem diesem Monitortyp zugeordneten Patientenkabel zu verbinden, das als Zubehör zu dem EKG-Monitor geliefert wird. Einer der eingangsseitigen Adapter des Patientenkabels wird mit dem hierzu passenden Ausgang des Universal-Adapters verbunden. Der Universal-Adapter stellt das Verbindungsglied zwischen der Eingangsleitung der rechten Schulter des monitorspezifischen Patientenkabels und dem Katheter dar. Die Ausgänge des Universal-Adapters sind so gestaltet, daß sie mit denjenigen Adapter zusammenpassen, die üblicherweise an den Enden von Patientenkabeln zum Verbinden mit den Haftelektroden vorhanden sind. Solche sind beispielsweise zangenartige Klemmenadapter, Druckknopfadapter oder Bananenstecker. Vorzugsweise sind an dem Universal-Adapter Ausgänge für diese drei genannten Konnektorarten vorgesehen. Der Universal-Adapter ermöglicht es somit, den Cava-Katheter mit unterschiedlichen Typen von Patientenkabeln zu verbinden. Er wird als Zubehörteil zu dem Cava-Katheter geliefert, an den er angeschlossen werden soll, und stellt die Verbindung mit unterschiedlichen Arten von monitorseitigen Patientenkabeln her.

Zwecks intraatrialer EKG-Ableitung zur Lageverifizierung eines Cava-Katheters wird die Eingangsleitung der rechten Schulter des Monitor-Patientenkabels mit dem Universal-Adapter verbunden. Bei einem Universal-Adapter gemäß Anspruch 2 wird die Eingangsleitung des Universal-Adapters dann mit der Brustwandelektrode der rechten Schulter verbunden bzw. bei einem Universal-Adapter nach Anspruch 1 direkt an den Katheter angeschlossen. Im ersten Fall ermöglicht ein Umschalter am Universal-Adapter das mühelose Wechseln vom extrakorporalen Brustwand-EKG auf intraatriales EKG, indem ein separates Verbindungskabel zwischen Universal-Adapter und Katheter konnektiert wird. Im zweiten Fall ist das Verbindungskabel als Eingangsleitung des Universal-Adapters ausgeführt, welches direkt an den Cava-Katheter angeschlossen wird. Ein Wechseln zwischen extrakorporalem EKG und intraatrialem EKG ist bei dieser Ausführung nur dann möglich, wenn die mit dem Universal-Adapter konnektierte Eingangsleitung der EKG-Patientenleitung gelöst wird und wieder mit der Brustwandelektrode der rechten Schulter verbunden wird.

In jedem Fall kann der Universal-Adapter in Verbindung mit zahlreichen Typen von Patientenkabeln benutzt werden, die sich auch durch die Anzahl ihrer Eingangsleitungen unterscheiden können. Der Lieferant des Cava-Katheters braucht nur den Universal-Adapter als Zubehörteil zum Cava-Katheter zu liefern, während das Patientenkabel das gerätespezifische Zubehörteil des EKG-Monitors darstellt. Der Adapter der Eingangsleitung für die rechte Schulter des Patientenkabels wird an einen Ausgang des Universal-Adapters angesteckt.

Im folgenden werden unter Bezugnahme auf die Zeichnungen Ausführungsbeispiele der Erfindung näher erläutert.

Es zeigen:
- Fig. 1: eine schematische Darstellung der Anschlußvorrichtung
- Fig. 2: einen schematischen Längsschnitt durch den Universal-Adapter nach Fig. 1
- Fig. 3: eine andere Ausführungsform des Adapters des Patientenkabels
- Fig. 4: eine weitere Ausführungsform des Adapters des Patientenkabels
- Fig. 5: eine schematische Darstellung einer Anschlußvorrichtung ohne Umschalter und
- Fig. 6: einen schematischen Längsschnitt durch den Universal-Adapter nach Fig. 5.

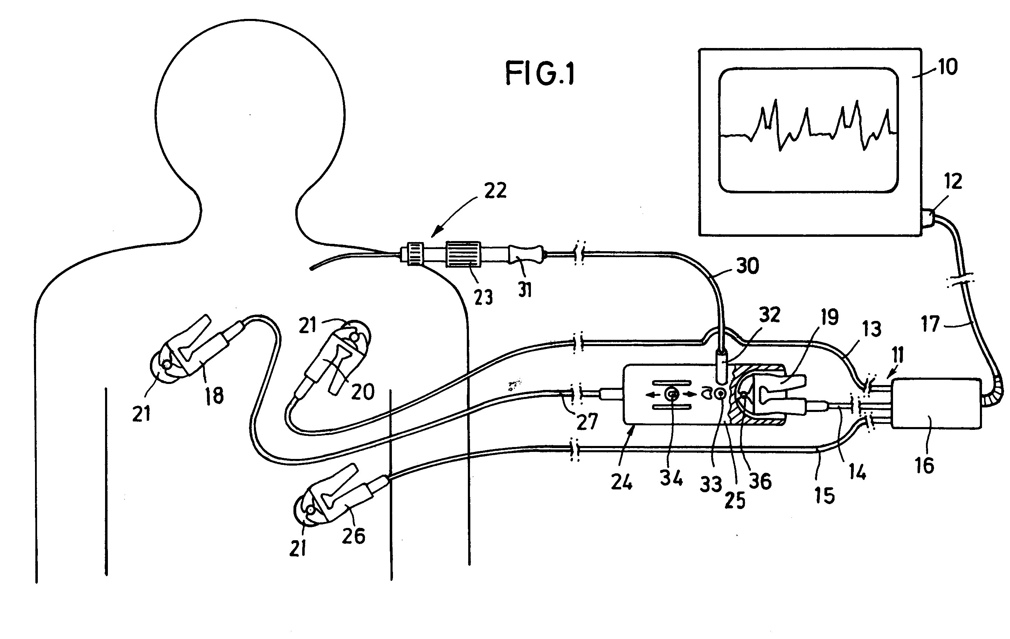

In Fig. 1 ist ein EKG-Monitor 10 nach Anspruch 2 dargestellt, mit dem EKG-Spannungen sichtbar gemacht bzw. aufgezeichnet werden können. An den Eingang des EKG-Monitors 10 ist das Patientenkabel 11 mit einem Vielfachstecker 12 angeschlossen. Der Vielfachstecker 12 des Patientenkabels ist auf den entsprechenden Gegenstecker bzw. die Steckerbuchse des EKG-Monitors 10 abgestimmt. Das Patientenkabel 11 weist bei dem vorliegenden Ausführungsbeispiel drei Leitungen 13,14,15 auf, die mit einem Verteiler 16 verbunden sind, von dem die mehradrige Leitung 17 zu dem Vielfachstecker 12 führt. Jede der Leitungen 13,14,15 weist an ihrem Ende einen Adapter 18, 19,20,26 auf, der hier als zangenartiger Klemmen-Adapter ausgebildet ist. Üblicherweise wird jeder der Adapter 19,20 und 26 an eine EKG-Elektrode 21 angeklemmt, die auf den Körper des Patienten befestigt wird.

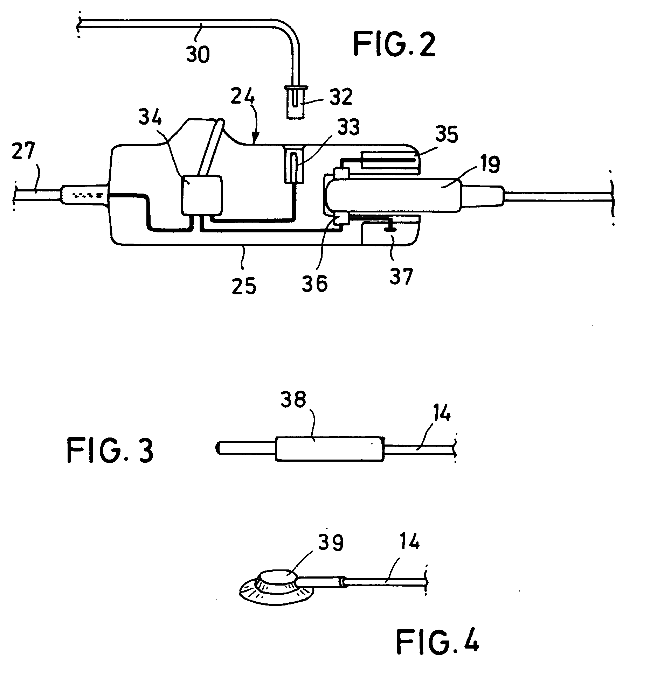

Ein Cava-Katheter 22 zur intraatrialen EKG-Ableitung ist mit einem Konnektor 23 versehen. Der erfindungsgemäße Universal-Adapter 24 dient dazu, den Cava-Katheter 22 mit dem zum EKG-Monitor 10 passenden Patientenkabel 11 zu verbinden. Er stellt ein Zusatzteil zum Herzkatheter 22 dar. Der Universal-Adapter weist ein Gehäuse 25 auf, in das der Adapter 18 der Eingangsleitung 27 hineinführt. Der Adapter 18 ist hier in gleicher Weise ausgebildet wie die Adapter 19,20 und 26, jedoch kann es sich auch um einen anderen Adaptertyp handeln (s. Fig. 3 u. 4), der mit einer EKG-Elektrode 21 verbindbar ist. In das Gehäuse 25 führt außerdem eine andere Eingangsleitung 30 hinein, die an einem Ende einen Katheter-Konnektor 31 aufweist, welcher mit dem Konnektor 23 des Cava-Katheters zusammenpaßt. Am anderen Ende der Leitung 30 befindet sich ein Stecker 32, der in eine Steckerfassung 33 des Gehäuses 24 eingesteckt werden kann.

Im Gehäuse 25 ist ein von außen zu betätigender Umschalter 34 angeordnet, mit dem entweder die eine Eingangsleitung 30 oder die andere Eingangsleitung 27 auf die Ausgänge des Universal-Adapters geschaltet werden kann.

Der Universal-Adapter 24 weist drei unterschiedlich gestaltete Ausgänge 35, 36 und 37 auf (s. Fig. 2), die für unterschiedliche Arten von Konnektoren geeignet sind. Der Ausgang 35 ist eine Bananenstecker-Fassung, in die der in Fig. 3 dargestellte Konnektor 38 in Form eines Bananensteckers eingesteckt werden kann. Der Ausgang 36 ist als Fassung für einen zangenartigen Klemmadapter 19 ausgebildet, wie er bei dem Ausführungsbeispiel von Fig. 1 eingesetzt ist. Der Ausgang 37 ist als Fassung für einen in Fig. 4 dargestellten Druckknopfkonnektor 39 ausgebildet. Generell sind die Fassungen 35, 36 und 37 von gleicher Art wie die Fassungen üblicher EKG-Elektroden, die auf der Haut des Patienten befestigt werden und mit denen die hier in Betracht kommenden Adapter zusammengreifen können. Die Ausgänge 35, 36 und 37 bzw. die betreffenden Fassungen sind so ausgebildet, daß sie den jeweils eingesetzten Adapter des passenden Typs mechanisch festhalten und elektrisch mit dem Ausgang des Umschalters 34 verbinden.

Die zu dem Universal-Adapter 24 gehörende Eingangsleitung 30 ist mit ihren Konnektoren 31 und 32 so ausgebildet, daß sie berührungssicher ist, d.h. daß die Leitungsader mit der Hand an keiner Stelle zugänglich ist.

Bei Benutzung der Anschlußvorrichtung werden die EKG-Elektroden 21 in bekannter Weise und Anordnung am Körper des Patienten befestigt. Dann werden die Konnektoren 20 und 26 (s. Fig. 1) des Patientenkabels 11 an die betreffenden Haftelektroden angeschlossen. Der Adapter 19 des Patientenkabels, der für die Ableitung des Brustwand-EKG bestimmt ist, wird in die passende Fassung des Universal-Adapters 24 eingesteckt, und anstelle dieses Adapters wird der Adapter 18 mit der EKG-Elektrode 21 für das Brustwand-EKG verbunden. Schließlich wird die Eingangsleitung 30 des Universal-Adapters mit dem Cava-Katheter 22 verbunden. Durch Betätigung des Umschalters 34 kann ausgewählt werden, ob die Eingangsleitung 27 oder die Eingangsleitung 30 über das Kabel 17 an den EKG-Monitor 10 angeschlossen wird. Die Leitungen 13 und 15 sind über den Verteiler 16 ständig mit dem EKG-Monitor verbunden.

Bei dem Ausführungsbeispiel der Fig. 1 sind die Adapter 19,20 und 26 des Patientenkabels 11 Klemmenadapter. Es besteht auch die Möglichkeit, ein Patientenkabel zu verwenden, das Adapter 38 in Form von Bananensteckern (Fig. 3) oder Adapter 39 in Form von Druckknöpfen aufweist.

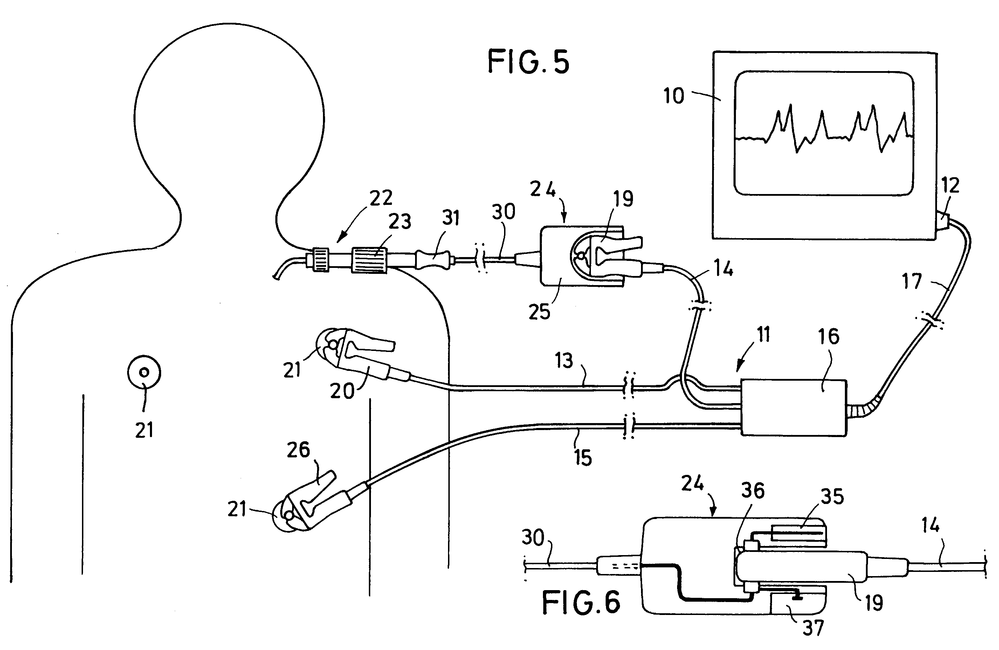

Bei dem Ausführungsbeispiel der Fig. 5 und 6 gemäß Anspruch 1 ist der Universal-Adapter 24 nicht mit einem Umschalter 34 versehen. Der Universal-Adapter hat hier die Eingangsleitung 30, die den Katheterkonnektor 31 aufweist. Diese Eingangsleitung 30 ist ständig mit den Ausgängen 35, 36 und 37 verbunden. Fig. 5 zeigt den Fall der intraatrialen EKG-Ableitung, wobei der Adapter 19 von Leitung 14 des Patientenkabels 11 in den Universal-Adapter 25 eingesteckt ist, so daß dem Monitor 10 die Spannung des Cava-Katheters über die Eingangsleitung 30 zugeführt wird. Wenn eine extrakorporale EKG-Ableitung durchgeführt werden soll, wird der Konnektor 19 aus dem Universal-Adapter 25 herausgezogen und an die betreffende freie EKG-Elektrode 21 (rechte Schulter) angesetzt.

## Patentansprüche

1. Anschlußvorrichtung zum Verbinden von EKG-Elektroden (21) mit einem Monitor (10), dem ein aus mehreren Leitungen bestehendes Patientenkabel (11) zugeordnet ist, welches an den Leitungsenden Adapter (19,20,26) für den Anschluß der Elektroden aufweist,
**gekennzeichnet durch**,
einen separaten Universal-Adapter (24), der an einer Eingangsleitung (30) einen mit einem Cava-Katheter (22) verbindbaren Katheter-Konnektor (31) aufweist und der mindestens zwei unterschiedlich gestaltete, wahlweise zu benutzende Ausgange (35,36,37) aufweist, die auf Adapter (19,38,39) unterschiedlichen Typs von Patientenkabeln (11) abgestimmt sind und die ständig mit der Eingangsleitung (30) verbunden sind.

2. Anschlußvorrichtung zum Verbinden von EKG-Elektroden (21) mit einem Monitor (10), dem ein aus mehreren Leitungen bestehendes Patientenkabel (11) zugeordnet ist, welches an den Leitungsenden Adapter (19,20,26) für den Anschluß der Elektroden aufweist,
**dadurch gekennzeichnet,**
daß ein separater Universal-Adapter (24) eine erste Eingangsleitung (30) mit einem mit einem Cava-Katheter (22) verbindbaren Katheter-Konnektor (31) und eine zweite Eingangsleitung (27) mit einem Adapter (18) für den Anschluß an eine extrakorporale Elektrode (21) aufweist, und daß der Universal-Adapter (24) mindestens zwei unterschiedlich gestaltete, wahlweise zu benutzende Ausgänge (35,36,37) aufweist, die auf Adapter (19,38,39) unterschiedlichen Typs von Patientenkabeln (11) abgestimmt sind und die mit einem im Universal-Adapter (24) enthaltenen Umschalter (34) entweder mit der ersten Eingangsleitung (30) oder mit der zweiten Eingangsleitung (27) verbindbar sind.

3. Anschlußvorrichtung nach Anspruch 1 oder 2, dadurch gekennzeichnet, daß ein Ausgang (36) des Universal-Adapters (24) als Fassung für einen zangenartigen Klemmadapter (19) ausgebildet ist.

4. Anschlußvorrichtung nach einem der Ansprüche 1 bis 3, dadurch gekennzeichnet, daß ein Ausgang (37) des Universal-Adapters (24) als Fassung für einen Druckknopfkonnektor (39) ausgebildet ist.

5. Anschlußvorrichtung nach einem der Ansprüche 1 bis 4, dadurch gekennzeichnet, daß ein Ausgang (35) des Universal-Adapters (24) als Bananenstecker-Fassung ausgebildet ist.

6. Anschlußvorrichtung nach einem der Ansprüche 1 bis 5, dadurch gekennzeichnet, daß der Universal-Adapter (24) ein Gehäuse (25) aufweist, in das die mindestens eine Eingangsleitung (30) hineinführt und das ausgangsseitig mehrere unterschiedliche Fassungen für unterschiedliche Adaptertypen (19,38 und 39) aufweist.

## Claims

1. A connecting device for connecting ECG electrodes (21) to a monitor (10), a patient cable (11) comprising a plurality of lines being associated with said monitor, said cable having adapters (19, 20, 26) at the ends of said lines for connecting said electrodes thereto,
characterized by
a separate universal adapter (24), which has an input line (30) provided with a catheter connector (31) connectable to a cava catheter (22) and which has at least two differently designed and selectively usable outputs (35, 36, 37) tailored to adapters (19. 38, 39) of different types of patient cables (11) and permanently connected with said input line (30).

2. A connecting device for connecting ECG electrodes (21) to a monitor (10), a patient cable (11) comprising a plurality of lines being associated with said monitor, said cable having adapters (19, 20, 26) at the ends of said lines for connecting said electrodes thereto,
characterized in
that a separate universal adapter (24) has a first input line (30) provided with a catheter connector (31) connectable to a cava catheter (22) and a second input line (27) with an adapter (18) for connection to an extracorporeal electrode (21), and that said universal adapter (24) has at least two differently designed and selectively usable outputs (35, 36, 37) tailored to adapters (19. 38, 39) of different types of patient cables (11) and which may be connected by means of a switch (34) within said universal adapter (24) either to said first input line (30) or said second input line (27).

3. The connecting device of claim 1 or 2, characterized in that one output (36) of the universal adapter (24) is designed as a socket for a tongs-like clamping adapter (19).

4. The connecting device of one of claims 1 to 3, characterized in that one output (37) of the universal adapter (24) is designed as a socket for a push-button connector (39).

5. The connecting device of one of claims 1 to 4, characterized in that one output (35) of the universal adapter (24) is designed as a socket for a banana plug.

6. The connecting device of one of claims 1 to 5, characterized in that said universal adapter (24) has a housing (25) into which extends said at least one input line (30) and which, on the output side, comprises a plurality of different sockets for different kinds of adapters (19, 38 and 39).

## Revendications

1. Dispositif de raccordement servant à relier des électrodes (21) d'enregistrement d'électrocardiogrammes à un moniteur (10), auquel est associé un câble (11) relié au patient et formé de plusieurs conducteurs et qui comporte, au niveau des extrémités des conducteurs, des adaptateurs (19,20,26) pour le raccordement des électrodes,
caractérisé par
un adaptateur universel séparé (24), qui comporte, au niveau d'un conducteur d'entrée (30), un connecteur de cathéter (31) pouvant être relié à un cathéter (22) placé dans la veine cave, et qui comporte au moins deux sorties (35,36,37) agencées différemment et qui peuvent être utilisées à volonté, lesquelles sont accordées sur des adaptateurs (19,38,39) de différents types de câbles (11) reliés au patient et sont reliées en permanence au conducteur d'entrée (30).

2. Dispositif de raccordement servant à relier des électrodes (21) d'enregistrement d'électrocardiogrammes à un moniteur (10), auquel est associé un câble (11) relié au patient et formé de plusieurs conducteurs et qui comporte, au niveau des extrémités des conducteurs, des adaptateurs (19,20,26) pour le raccordement des électrodes,
caractérisé en ce
qu'un adaptateur universel séparé (24) possède un premier conducteur d'entrée (30) comportant un connecteur de cathéter (31) pouvant être relié à un cathéter (22) placé dans la veine cave, et un second conducteur d'entrée (27) comportant un adaptateur (18) pour le raccordement à une électrode extra-corporelle (21) et que l'adaptateur universel (24) comporte au moins deux sorties (35,36,37) agencées différemment, pouvant être utilisées à volonté, et qui sont accordées sur des adaptateurs (19,38,39) de différents types de câbles (11) reliés au patient et qui peuvent être reliées à un commutateur (34) contenu dans l'adaptateur universel (24), soit au premier conducteur d'entrée (30), soit au second conducteur d'entrée (27).

3. Dispositif de raccordement selon la revendication 1 ou 2, caractérisé en ce qu'une sortie (36) de l'adaptateur universel (24) est agencée en tant que monture pour un adaptateur en forme de pince (19).

4. Dispositif de raccordement selon l'une des revendications 1 à 3, caractérisé en ce qu'une sortie (37) de l'adaptateur universel (24) est agencée en tant que monture pour un connecteur à bouton-poussoir (39).

5. Dispositif de raccordement selon l'une des revendications 1 à 4, caractérisé en ce qu'une sortie (35) de l'adaptateur universel (24) est agencée en tant que monture de connecteur du type banane.

6. Dispositif de raccordement selon l'une des revendications 1 à 5, caractérisé en ce que l'adaptateur universel (24) comporte un boîtier (25) dans lequel est introduit au moins un conducteur d'entrée (30) et qui comporte, côté sortie, plusieurs montures différentes pour différents types d'adaptateurs (19,38 et 39).
